# EUROPEAN PATENT APPLICATION

(11) **EP 4 745 154 A1**
(43) Date of publication of application: **20.05.2026**
(21) Application number: 24867246.1
(22) Date of filing: 27.08.2024
(51) Int. Cl.: C07K 14/725, C12N 15/12, C12N 15/861, C12N 15/864, C12N 15/867, C12N 7/01, C12N 5/10, A61K 39/00, A61P 35/00, A61P 35/02

(54) **ISOLATED TCR AND USE THEREOF**

(30) Priority: 22.09.2023 CN 202311231500; 22.08.2024 CN 202411160424
(71) Applicant: Shanghai General Hospital, Shanghai 200080 (CN)
(72) Inventor: SHEN, Lianghua, Shanghai 200080 (CN); WANG, Pengran, Shanghai 200080 (CN); DING, Xiaodan, Shanghai 200080 (CN); XU, Jian, Shanghai 200080 (CN); MA, Ling, Shanghai 200080 (CN); ZHANG, Yan, Shanghai 200080 (CN); SONG, Xianmin, Shanghai 200080 (CN)
(74) Representative: Canzler & Bergmeier Patentanwälte Partnerschaft mbB
(86) International application number: PCT/CN2024/114800
(87) International publication number: WO 2025/060832

(57) **Abstract**

The present invention relates to the field of biomedicine, and in particular to an isolated TCR and a use thereof. The TCR comprises a TCR α chain and a TCR β chain; the TCR α chain comprises CDRα1, CDRα2 and CDRα3 in a TCR α chain variable region; the TCR β chain comprises CDRβ1, CDRβ2 and CDRβ3 in a TCR β chain variable region; the amino acid sequence of CDRα3 is as shown in SEQ ID NO. 2; and the amino acid sequence of CDRβ3 is as shown in SEQ ID NO. 1. A TP53-R248Q-TCR-T cell prepared by stably expressing TP53-R248Q-TCR in a CD8⁺ T cell can specifically recognize and kill a tumor cell which expresses HLA-A*11:01 and a TP53-R248Q mutant antigen. A solid theoretical and experimental foundation is provided for developing TCR-T therapy targeting a TP53-R248Q tumor neoantigen.

## Description

### FIELD OF TECHNOLOGY

The present disclosure relates to the technical field of biomedicine, and in particular to an isolated T cell receptor (TCR) and a use thereof.

### BACKGROUND

p53 is a well-known tumor suppressor protein that regulates the cell cycle and prevents malignant transformation of cells. It is known as the "guardian of the genome", and its corresponding TP53 gene is one of the most frequently mutated tumor suppressor genes in human cancers. TP53-R248Q, which ranks second among high-frequency mutations in malignant tumors, is closely associated with disease relapse, poor therapeutic response, and drug resistance during patient treatment. Mutations in the p53 protein occur in approximately 50% of cancers, while in the remaining cancers, alternative mechanisms are frequently employed to promote functional inactivation of p53. Missense mutations are generally classified into two categories. One category includes DNA-contact mutations, which occur at amino acid residues that directly interact with DNA, resulting in the inability of p53 to bind DNA, such as p53 R273H and R248Q mutants. The other category includes conformational mutations, which occur at amino acid residues responsible for maintaining protein structure, leading to protein misfolding, such as p53 R175H, Y220C, and R249S mutants. Studies have shown that acquired TP53-R248Q mutations are associated with disease relapse during treatment, poor therapeutic responses, and resistance to multiple chemotherapeutic agents in patients. Further research indicated that the TP53-R248Q mutation not only resulted in loss of tumor suppressor function but also acted as a gain-of-function mutation that promotes tumorigenesis in mice. In addition, TP53-R248Q mutations have been shown to increase invasive behavior in cell lines and are associated with poorer overall survival outcomes. Therefore, targeting TP53-R248Q tumor neoantigens is of great significance for cellular therapies towards multiple tumors carrying this mutation.

In recent years, immune cell therapy has emerged as a novel tumor treatment modality following surgery, radiotherapy, chemotherapy, small-molecule targeted therapy, and monoclonal antibody therapy. TCR-T therapy involves the use of genetic engineering to introduce cloned TCRs that specifically recognize tumor antigens onto autologous CD8⁺ T cells, thereby conferring tumor-specific cytotoxic activity. Compared with CAR-T cells, which can only recognize membrane antigens, TCR-T cells recognize antigenic peptides presented by major histocompatibility complex (MHC) molecules. Thus, TCR-T cells can target a broader range of antigens, including both intracellular and membrane antigens. TCR-T cell therapy not only increases the number of T lymphocytes but also enhances their tumor-specific cytotoxicity, thereby achieving improved antitumor effects. Recent clinical studies have demonstrated the favorable therapeutic effects of TCR-T therapy in tumor patients. However, due to the individual specificity of tumor-specific antigens and the diversity of the TCR repertoire, obtaining TCR sequences that specifically recognize tumor neoantigens remains highly challenging.

### SUMMARY

The present disclosure provides an isolated TCR and a use thereof, which can address the problems existing in the prior art.

The isolated TCR of the present disclosure includes a TCR α chain and a TCR β chain. The TCR α chain includes CDR α1, CDR α2, and CDR α3 in a variable region of the TCR α chain. The TCR β chain includes CDR β1, CDR β2, and CDR β3 in a variable region of the TCR β chain. CDR α3 includes an amino acid sequence as set forth in SEQ ID NO: 2, and CDR β3 includes an amino acid sequence as set forth in SEQ ID NO: 1.

In some embodiments, the CDR α1 includes an amino acid sequence as set forth in SEQ ID NO: 3, or the CDR α2 includes an amino acid sequence as set forth in SEQ ID NO: 4; and/or the CDR β1 includes an amino acid sequence as set forth in SEQ ID NO: 5, or the CDR β2 includes an amino acid sequence as set forth in SEQ ID NO: 6.

The present disclosure further provides a polynucleotide encoding the TCR described above.

The present disclosure further provides a nucleic acid construct, including the polynucleotide described above and a plasmid backbone.

The present disclosure further provides a virus, including the polynucleotide described above.

The present disclosure further provides a T cell, including the TCR, the polynucleotide, the nucleic acid construct, or the virus described above.

The present disclosure further provides a use of the TCR, the polynucleotide, the nucleic acid construct, the virus, or the T cell described above in the preparation of a tumor therapeutic product.

The present disclosure further provides a tumor therapeutic product, including the TCR, the polynucleotide, the nucleic acid construct, the virus, or the T cell described above, and a pharmaceutically acceptable carrier.

The present disclosure further provides a method for treating a tumor, including administering to a subject in need thereof the TCR, the polynucleotide, the nucleic acid construct, the virus, or the T cell described above.

The isolated TCR and a use thereof provided by the present disclosure have the following beneficial effects:

Based on the population distribution of HLA subtypes and the mutation frequency of TP53 mutants, a high-prevalence HLA subtype (HLA-A*11:01) was selected, and under this HLA restriction, a high-frequency TP53-R248Q (Rank 2#) tumor-specific antigen (TSA) was selected. TP53-R248Q tumor antigen-specific TCRs were screened *in vitro,* followed by single-cell TCR sequencing to obtain TCR sequences, and comprehensive validation of TP53-R248Q-TCR specificity and function was performed. TP53-R248Q-TCR-T cells were prepared by stably expressing the TP53-R248Q-TCR of the present disclosure on CD8⁺ T cells. These TP53-R248Q-TCR-T cells specifically recognized and killed tumor cells expressing HLA-A*11:01 and tumor cells expressing the TP53-R248Q mutant antigen in both *in vitro* and *in vivo* experiments, had significantly enhanced IFN-γ release, and exhibited potent antigen-specific cytotoxicity against target cells. The TCR and TCR-T cells described herein can be used in the development of TCR-T therapies targeting TP53-R248Q tumor-specific antigens.

### BRIEF DESCRIPTION OF THE DRAWINGS

FIG. 1 shows the sorting and single-cell sequencing results of the TP53-R248Q-TCR-T cells of the present disclosure.
FIG. 2 shows the *in vitro* binding assay results of the TP53-R248Q-TCR-T cells of the present disclosure.
FIG. 3 shows the *in vitro* activation assay results of the TP53-R248Q-TCR-T cells of the present disclosure.
FIG. 4 shows the *in vitro* cytotoxicity assay results of the TP53-R248Q-TCR-T cells of the present disclosure.
FIG. 5 shows the *in vivo* cytotoxicity assay results of the TP53-R248Q-TCR-T cells of the present disclosure.

### DETAILED DESCRIPTION

The present disclosure provides an isolated T cell receptor (TCR) or a fragment thereof. The TCR includes a TCR α chain and a TCR β chain. The TCR α chain includes a variable region of the TCR α chain and a constant region of the TCR α chain. The TCR β chain includes a variable region of the TCR β chain and a constant region of the TCR β chain. The TCR α chain includes CDR α1, CDR α2, and CDR α3 within the variable region of the TCR α chain. The TCR β chain includes CDR β1, CDR β2, and CDR β3 within the variable region of the TCR β chain. The CDR α3 includes an amino acid sequence as set forth in SEQ ID NO: 2, and the CDR β3 includes an amino acid sequence as set forth in SEQ ID NO: 1.

In some embodiments, the CDR α1 includes an amino acid sequence as set forth in SEQ ID NO: 3, or the CDR α2 includes an amino acid sequence as set forth in SEQ ID NO: 4.

In some embodiments, the CDR β1 includes an amino acid sequence as set forth in SEQ ID NO: 5, or the CDR β2 includes an amino acid sequence as set forth in SEQ ID NO: 6.

The term "complementarity determining region" (CDR) generally refers to a region within an antibody or a receptor that is spatially capable of forming complementary interactions with an antigenic determinant. Variability of an antibody or a receptor is typically not evenly distributed throughout the entire variable region. The variable regions of a monoclonal antibody or the variable regions of the α chain and the β chain of a receptor typically each include three hypervariable regions (HVRs). These HVRs are spatially capable of forming complementary interactions with antigenic determinants and are therefore also referred to as complementarity determining regions (CDRs). Accordingly, the variable region of the α chain typically includes three CDRs, namely CDR α1, CDR α2, and CDR α3, and the variable region of the β chain typically also includes three CDRs, namely CDR β1, CDR β2, and CDR β3.

In some embodiments, the variable regions of the α chain and the β chain may further include framework regions (FRs). The FRs may be located between the CDRs or at both ends of the CDRs. In certain embodiments, the FR sequence is obtained by substitution, deletion, or addition of one or more amino acids (for example, 1-50, 1-30, 1-20, 1-10, 1-5, or 1-3 amino acids) based on a FR sequence of a human monoclonal antibody variable region or a murine monoclonal antibody variable region, which may have at least 80%, 85%, 90%, 93%, 95%, 97%, or 99% sequence homology with the FR sequence of a human monoclonal antibody variable region.

In some embodiments, the TCR α chain further includes a TCR α chain constant region, and the TCR β chain further includes a TCR β chain constant region.

In some embodiments, the variable region of the TCR α chain includes an amino acid sequence as set forth in SEQ ID NO: 14; and/or the variable region of the TCR β chain includes an amino acid sequence as set forth in SEQ ID NO: 15.

In some embodiments, the variable region of the TCR α chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 14. The variable region of the TCR β chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 15.

In some embodiments, the constant region of the TCR α chain includes an amino acid sequence as set forth in SEQ ID NO: 11, and the constant region of the TCR β chain includes an amino acid sequence as set forth in SEQ ID NO: 10.

In some embodiments, the constant region of the TCR α chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 11. The constant region of the TCR β chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 10.

In some embodiments, the TCR α chain includes an amino acid sequence as set forth in SEQ ID NO: 8, and the TCR β chain includes an amino acid sequence as set forth in SEQ ID NO: 7.

In some embodiments, provided that the above α-chain and β-chain variable region CDR sequences are present, the TCR α chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 8; and the TCR β chain may be a peptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the amino acid sequence as set forth in SEQ ID NO: 7.

In some embodiments, the TCR α chain is a human TCR α chain, a humanized TCR α chain, a chimeric TCR α chain, or a murine TCR α chain. The TCR β chain is a human TCR β chain, a humanized TCR β chain, a chimeric TCR β chain, or a murine TCR β chain. As used herein, the term "chimeric TCR α chain" or "chimeric TCR β chain" refers to a TCR α chain or TCR β chain including sequences derived from more than one species, such as sequences derived from human and mouse.

In some embodiments, the TCR binds to a p53 antigen. Preferably, the TCR binds to a TP53-R248Q antigen.

The present disclosure further provides an antigen-binding protein, which includes the TCR described above.

The present disclosure further provides a polynucleotide, which encodes the TCR described above.

In some embodiments, the polynucleotide includes a nucleotide sequence as set forth in SEQ ID NO: 9.

In some embodiments, the nucleotide sequence of the polynucleotide has at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 99.5% sequence identity to the nucleotide sequence as set forth in SEQ ID NO: 9.

The present disclosure further provides a nucleic acid construct, including the polynucleotide described above and a plasmid backbone.

In some embodiments, the plasmid backbone is a plasmid backbone of a virus, an adenovirus, or an adeno-associated virus. Specifically, the plasmid backbone is one or more of pLKO.1-CMV-tGFP, pLKO.1-puro-CMV-tGFP, pLKO.1-CMV-Neo, pLKO.1-Neo, pLKO.1-Neo-CMV-tGFP, pLKO.1-puro-CMV-TagCFP, pLKO.1-puro-CMV-TagYFP, pLKO.1-puro-CMV-TagRFP, pLKO.1-puro-CMV-TagFP635, pLKO.1-puro-UbC-TurboGFP, pLKO.1-puro-UbC-TagFP635, pLKO-puro-IPTG-1xLacO, pLKO-puro-IPTG-3xLacO, pLP1, pLP2, pLP/VSV-G, pENTR/U6, pLenti6/BLOCK-iT-DEST, pcDNA1.2/V5-GW/lacZ, pLenti6.2/N-Lumio/V5-DEST, pGCSIL-GFP, and pHR-SFFV-Puro.

The present disclosure provides a virus, which includes the polynucleotide described above.

In some embodiments, the virus is a lentivirus, an adenovirus, or an adeno-associated virus.

The present disclosure provides a T cell, which includes the TCR, the polynucleotide, the nucleic acid construct, or the virus described above.

In some embodiments, the T cell is a TCR-T cell.

In some embodiments, the T cell is a helper T cell, a suppressor T cell, an effector T cell, a cytotoxic T cell, a delayed-type hypersensitivity T cell, a natural T cell, or a memory T cell. Helper T cells are characterized by functions of assisting humoral and cellular immunity. Suppressor T cells function to suppress humoral and cellular immune responses. Effector T cells are capable of releasing lymphokines. Cytotoxic T cells are capable of killing target cells. Delayed-type hypersensitivity T cells are involved in type IV hypersensitivity reactions and can act on helper T cells and suppressor T cells to enhance immune effects. Natural T cells are undifferentiated T cells. Memory T cells are characterized by the ability to retain antigen specificity and immunological memory upon re-encounter with the same (or closely related) antigen.

In some embodiments, the T cell is a Jurkat T cell or a CD8⁺ T cell.

The present disclosure further provides a use of the TCR, the polynucleotide, the nucleic acid construct, the virus, or the T cell described above in the preparation of a tumor therapeutic product.

In some embodiments, the tumor is one or more of adrenocortical carcinoma, urothelial carcinoma (bladder cancer), breast cancer, cervical squamous cell carcinoma, cervical adenocarcinoma, cholangiocarcinoma, colon adenocarcinoma, lymphoid neoplasm, esophageal carcinoma, glioblastoma multiforme, head and neck squamous cell carcinoma, chromophobe renal cell carcinoma, clear cell renal cell carcinoma, papillary renal cell carcinoma, acute myeloid leukemia, low-grade glioma, hepatocellular carcinoma, mesothelioma, ovarian cancer, pancreatic cancer, pheochromocytoma, paraganglioma, prostate cancer, rectal cancer, sarcoma, melanoma, gastric cancer, testicular germ cell tumor, thyroid carcinoma, thymic carcinoma, endometrial carcinoma, chronic myeloid leukemia, lung cancer, anal cancer, and retinoblastoma.

The present disclosure further provides a tumor therapeutic product, which includes the TCR, the polynucleotide, the nucleic acid construct, the virus, or the T cell described above, and a pharmaceutically acceptable carrier.

In some embodiments, the carrier includes various excipients and diluents, which are not necessary active ingredients and do not exhibit excessive toxicity after administration. The carrier may include sterile water or physiological saline, stabilizers, excipients, antioxidants (such as ascorbic acid), buffers (such as phosphoric acid, citric acid, or other organic acids), preservatives, surfactants (such as PEG or Tween^{®}), chelating agents (such as EDTA), or adhesives. The carrier may further include other low-molecular-weight polypeptides, serum albumin, glycine, glutamine, asparagine, arginine, polysaccharides, monosaccharides, mannitol, or sorbitol. When the carrier is an aqueous solution for injection, it may be selected from physiological saline, isotonic glucose solutions, isotonic D-sorbitol solutions, isotonic D-mannose solutions, and isotonic D-mannitol solutions. The aqueous solution for injection includes a solubilizer. The solubilizer is selected from alcohols (such as ethanol), polyols (such as propylene glycol or PEG), and nonionic surfactants (such as Tween^{®} 80 or HCO-50). In the tumor therapeutic product of the present disclosure, the TCR, the polynucleotide, the nucleic acid construct, or the T cell described above may be used as a single active ingredient, or may be combined with one or more other active components to form a combination formulation. The active components are other drugs used for tumor treatment. The content of the active components in the tumor therapeutic product is a safe and effective amount, which can be adjusted by those skilled in the art. For example, the administered amount of the TCR, the polynucleotide, the nucleic acid construct, or the T cell described above, and the active ingredients of the tumor therapeutic product depend on factors such as the patient's body weight, the type of administration, and the condition and severity of the disease. For example, the dose of the bifunctional compound as the active ingredient is in a range of 1-1000 mg/kg/day, 1-3 mg/kg/day, 3-5 mg/kg/day, 5-10 mg/kg/day, 10-20 mg/kg/day, 20-30 mg/kg/day, 30-40 mg/kg/day, 40-60 mg/kg/day, 60-80 mg/kg/day, 80-100 mg/kg/day, 100-200 mg/kg/day, 200-500 mg/kg/day, or greater than 500 mg/kg/day.

The present disclosure further provides a method for treating a tumor, which includes administering to a subject in need thereof an effective amount of the TCR, the polynucleotide, the nucleic acid construct, the virus, or the T cell described above.

In some embodiments, the administered amount is in a range of 1-1000 mg/kg/day. Specifically, the administered amount is in a range of 1-3 mg/kg/day, 3-5 mg/kg/day, 5-10 mg/kg/day, 10-20 mg/kg/day, 20-30 mg/kg/day, 30-40 mg/kg/day, 40-60 mg/kg/day, 60-80 mg/kg/day, 80-100 mg/kg/day, 100-200 mg/kg/day, 200-500 mg/kg/day, or 500-1000 mg/kg/day.

In some embodiments, the subject is a mammal; preferably, the subject is a human.

In some embodiments, the tumor is a tumor in which the wild-type TP53 gene harbors an R248Q mutation. Information regarding the specific nucleotide sequence or amino acid sequence of the wild-type TP53 gene can be obtained from existing databases, such as NCBI or Ensembl.

In the present disclosure, the T cell receptor (TCR) is a molecule present on the surface of T cells and is responsible for recognizing peptide-MHC complexes. In some embodiments, the TCR is a truncated or a full-length TCR. In some embodiments, the TCR is a heterodimer including an α chain and a β chain. In some embodiments, the TCR may be a single-chain TCR (scTCR).

In the present disclosure, the term "TCR α chain constant region" or "TCR β chain constant region" includes, in sequence, an extracellular constant region, a transmembrane region, and an intracellular constant region. The extracellular constant region may include hinge regions of both the TCR α chain and the TCR β chain, and it participates in the formation of disulfide bonds between the TCR α chain and the TCR β chain. The transmembrane region, which is part of the constant region, functions to anchor the TCR α chain and the TCR β chain to the cell membrane, and to participate in the interaction between the TCR α and β chains and the CD3 subunits, thereby helping to form the TCR-CD3 complex. The intracellular constant region may function to participate in the conformational change and signal transduction of the TCR-CD3 complex following TCR signal transduction.

In the present disclosure, the term "variable region" or "variable domain" refers to a domain of an immunoglobulin superfamily binding protein (for example, TCR α chains or β chains, or TCR γ chains or δ chains) that participates in binding between an immunoglobulin superfamily binding protein (for example, a TCR) and an antigen. The variable domains of the α chain and β chain of a natural TCR (designated Vα and Vβ, respectively) typically have similar structures, each domain including four conserved FRs and three CDRs. The Vα domain is encoded by two separate DNA segments, namely a variable gene segment and a joining gene segment (VJ). The Vβ domain is encoded by three separate DNA segments, namely a variable gene segment, a diversity gene segment, and a joining gene segment (VDJ). A single Vα or Vβ domain may be sufficient to confer antigen-binding specificity. In addition, for a TCR that binds a specific antigen, the Vα or Vβ domain can be isolated from the antigen-binding TCR to construct and screen libraries of complementary Vα or Vβ domains, respectively.

In the present disclosure, the term "antigen" refers to a molecule that is present on the cell surface or presented intracellularly by an MHC molecule or an MHC-like molecule and can be bound by an antibody or a T cell receptor (TCR). Examples of antigens include, but are not limited to, polypeptide antigens (such as NYESO-1, AFP, and MART-1), lipid antigens (such as β-GlcCer, eLPA, and LPE), and polysaccharide antigens (such as CA199, CA72-4, and CA125). The antigen may be a tumor antigen, such as a tumor-associated antigen (TAA) or a tumor-specific antigen (TSA).

In the present disclosure, the term "isolated" refers to a material that has been removed from its natural state or otherwise subjected to human manipulation, such as the α chain, β chain, T cell receptor, and nucleic acids described herein. An isolated material may be essentially or substantially free of components that normally accompany it in its natural state, or may be manipulated to be in an artificial state while remaining associated with components that normally accompany it in its natural state. An isolated material may be in a natural, chemically synthesized, or recombinant form. Alternatively, an isolated material may be in an enriched, partially purified, or purified form.

In the present disclosure, the term "polynucleotide", also referred to as "nucleotide" or "nucleic acid", refers to deoxyribonucleic acid, ribonucleic acid, modified nucleic acids or bases, and/or analogs thereof, or a nucleic acid chain composed of any substrate that can be incorporated into a chain by a DNA or RNA polymerase.

In the present disclosure, the term "nucleic acid construct", also referred to as a "vector", refers to a construct capable of delivering one or more target genes or sequences into a host cell and preferably expressing the genes or sequences in the host cell. Examples of vectors include, but are not limited to, viral vectors, plasmids, cosmids, or bacteriophage vectors.

In the present disclosure, the term "host cell" refers to a cell into which exogenous nucleic acid has been introduced, including all progeny derived from such a cell.

In the present disclosure, to determine the percentage identity between two amino acid sequences or two nucleic acid sequences, the sequences are aligned for optimal comparison purposes (for example, gaps may be introduced into one or both of the first and second amino acid sequences or nucleic acid sequences for optimal alignment, or non-homologous sequences may be discarded for comparison purposes). In one embodiment, for comparison purposes, the length of the aligned reference sequence is at least 30%, preferably at least 40%, more preferably at least 50%, at least 60%, and even more preferably at least 70%, at least 80%, at least 90%, or at least 100% of the length of the reference sequence. The amino acid residues or nucleotides at corresponding amino acid or nucleotide positions are then compared. When a position in the first sequence is occupied by the same amino acid residue or nucleotide as the corresponding position in the second sequence, the molecules are identical at that position.

Sequence comparison and calculation of the percentage identity between two sequences may be performed using mathematical algorithms. In one embodiment, the percent identity between two amino acid sequences is determined using the Needleman and Wunsch algorithm ((1970) J. Mol. Biol. 48:444-453) implemented in the GAP program of the GCG software package (available at http://www.gcg.com), using a Blossum 62 matrix or a PAM250 matrix with a gap weight of 16, 14, 12, 10, 8, 6, or 4, and a length weight of 1, 2, 3, 4, 5, or 6. In another embodiment, the percent identity between two amino acid sequences is determined using the GAP program of the GCG software package (available at http://www.gcg.com) , using the NWSgapdna.CMP matrix, with a gap weight of 40, 50, 60, 70, or 80, and a length weight of 1, 2, 3, 4, 5, or 6. A particularly preferred set of parameters, which should be used unless otherwise specified, employs the Blossum 62 scoring matrix with a gap opening penalty of 12, a gap extension penalty of 4, and a frameshift gap penalty of 5.

Alternatively, percent identity between two amino acid sequences or two nucleic acid sequences can also be determined using the E. Meyers and W. Miller algorithm ((1989) CABIOS 4:11-17) incorporated into the ALIGN program (Version 2.0), using the PAM120 weighted residue table, with a gap length penalty of 12, and a gap penalty of 4.

The following specific examples are provided to illustrate embodiments of the present disclosure. Those skilled in the art will readily understand other advantages and effects of the present disclosure from the disclosure herein. The present disclosure may also be implemented or applied through other different specific embodiments, and various modifications or changes may be made to the details of the present specification based on different viewpoints and applications without departing from the scope of the present disclosure.

Before further describing specific embodiments of the present disclosure, it should be understood that the scope of protection of the present disclosure is not limited to the specific embodiments described below. It should also be understood that the terminology used in the embodiments of the present disclosure is for the purpose of describing particular embodiments only and is not intended to limit the scope of the present disclosure. In the present disclosure, unless otherwise explicitly indicated, the singular forms "a," "an," and "the" include the plural forms.

When numerical ranges are provided in the examples, it should be understood that, unless otherwise specified, both endpoints of each numerical range and any value between the two endpoints may be selected. Unless otherwise defined, all technical and scientific terms used herein have the same meanings as commonly understood by those skilled in the art. In addition to the specific methods, devices, and materials used in the examples, any methods, devices, or materials of the prior art that are similar or equivalent to those described in the embodiments of the present disclosure may also be used to implement the present disclosure, based on the knowledge of those skilled in the art and the teachings of the present disclosure.

The sequence information in the present disclosure is as follows:
SEQ ID NO:1
   CASSDDTGANYGYTF
SEQ ID NO:2
   CAPDNAGNMLTF
SEQ ID NO:3
   NYSPAY
SEQ ID NO:4
   IRENEKE
SEQ ID NO:5
   SNHLY
SEQ ID NO:6
   FYNNEI
SEQ ID NO:7 
MDTWLVCWAIFSLLKAGLTEPEVTQTPSHQVTQMGQEVILRCVPISNHLY
FYWYRQILGQKVEFLVSFYNNEISEKSEIFDDQFSVERPDGSNFTLKIRSTKLEDS
AMYFCASSDDTGANYGYTFGSGTRLTVVEDLRNVTPPKVSLFEPSKAEIANKQK
ATLVCLARGFFPDHVELSWWVNGKEVHSGVCTDPQAYKESNYSYCLSSRLRVS
ATFWHNPRNHFRCQVQFHGLSEEDKWPEGSPKPVTQNISAEAWGRADCGITS
ASYQQGVLSATILYEILLGKATLYAVLVSTLVVMAMVKRKNS
SEQ ID NO:8 
MESFLGGVLLILWLQVDWVKSQKIEQNSEALNIQEGKTATLTCNYTNYSP
AYLQWYRQDPGRGPVFLLLIRENEKEKRKERLKVTFDTTLKQSLFHITASQPADS
ATYLCAPDNAGNMLTFGGGTRLMVKPHIQNPEPAVYQLKDPRSQDSTLCLFTDF
DSQINVPKTMESGTFITDKCVLDMKAMDSKSNGAIAWSNQTSFTCQDIFKETNAT
YPSSDVPCDATLTEKSFETDMNLNFQNLSVMGLRILLLKVAGFNLLMTLRLWSS
SEQ ID NO:9 
ATGGAGAGCTTCCTGGGCGGCGTGCTGCTGATCCTGTGGCTGCAGGT
GGACTGGGTGAAGAGCCAGAAGATCGAGCAGAACAGCGAGGCCCTGAACAT
CCAGGAGGGCAAGACCGCCACCCTGACCTGCAACTACACCAACTACAGCCC
CGCCTACCTGCAGTGGTACAGGCAGGACCCCGGCAGGGGCCCCGTGTTCC
TGCTGCTGATCAGGGAGAACGAGAAGGAGAAGAGGAAGGAGAGGCTGAAG
GTGACCTTCGACACCACCCTGAAGCAGAGCCTGTTCCACATCACCGCCAGC
CAGCCCGCCGACAGCGCCACCTACCTGTGCGCCCCCGACAACGCCGGCAA
CATGCTGACCTTCGGCGGCGGCACCAGGCTGATGGTGAAGCCCCACATCCA
GAACCCCGAGCCCGCCGTGTACCAGCTGAAGGACCCCAGGAGCCAGGACA
GCACCCTGTGCCTGTTCACCGACTTCGACAGCCAGATCAACGTGCCCAAGA
CCATGGAGAGCGGCACCTTCATCACCGACAAGTGCGTGCTGGACATGAAGG
CCATGGACAGCAAGAGCAACGGCGCCATCGCCTGGAGCAACCAGACCAGCT
TCACCTGCCAGGACATCTTCAAGGAGACCAACGCCACCTACCCCAGCAGCG
ACGTGCCCTGCGACGCCACCCTGACCGAGAAGAGCTTCGAGACCGACATGA
ACCTGAACTTCCAGAACCTGAGCGTGATGGGCCTGAGGATCCTGCTGCTGA
AGGTGGCCGGCTTCAACCTGCTGATGACCCTGAGGCTGTGGAGCAGCATGG
ACACCTGGCTGGTGTGCTGGGCCATCTTCAGCCTGCTGAAGGCCGGCCTGA
CCGAGCCCGAGGTGACCCAGACCCCCAGCCACCAGGTGACCCAGATGGGC
CAGGAGGTGATCCTGAGGTGCGTGCCCATCAGCAACCACCTGTACTTCTACT
GGTACAGGCAGATCCTGGGCCAGAAGGTGGAGTTCCTGGTGAGCTTCTACA
ACAACGAGATCAGCGAGAAGAGCGAGATCTTCGACGACCAGTTCAGCGTGG
AGAGGCCCGACGGCAGCAACTTCACCCTGAAGATCAGGAGCACCAAGCTGG
AGGACAGCGCCATGTACTTCTGCGCCAGCAGCGACGACACCGGCGCCAACT
ACGGCTACACCTTCGGCAGCGGCACCAGGCTGACCGTGGTGGAGGACCTG
AGGAACGTGACCCCCCCCAAGGTGAGCCTGTTCGAGCCCAGCAAGGCCGA
GATCGCCAACAAGCAGAAGGCCACCCTGGTGTGCCTGGCCAGGGGCTTCTT
CCCCGACCACGTGGAGCTGAGCTGGTGGGTGAACGGCAAGGAGGTGCACA
GCGGCGTGTGCACCGACCCCCAGGCCTACAAGGAGAGCAACTACAGCTACT
GCCTGAGCAGCAGGCTGAGGGTGAGCGCCACCTTCTGGCACAACCCCAGG
AACCACTTCAGGTGCCAGGTGCAGTTCCACGGCCTGAGCGAGGAGGACAAG
TGGCCCGAGGGCAGCCCCAAGCCCGTGACCCAGAACATCAGCGCCGAGGC
CTGGGGCAGGGCCGACTGCGGCATCACCAGCGCCAGCTACCAGCAGGGCG
TGCTGAGCGCCACCATCCTGTACGAGATCCTGCTGGGCAAGGCCACCCTGT
ACGCCGTGCTGGTGAGCACCCTGGTGGTGATGGCCATGGTGAAGAGGAAGA
ACAGC
SEQ ID NO:10 
EDLRNVTPPKVSLFEPSKAEIANKQKATLVCLARGFFPDHVELSWWVNG
KEVHSGVCTDPQAYKESNYSYCLSSRLRVSATFWHNPRNHFRCQVQFHGLSEE
DKWPEGSPKPVTQNISAEAWGRADCGITSASYQQGVLSATILYEILLGKATLYAV
LVSTLVVMAMVKRKNS
SEQ ID NO:11 
DIQNPEPAVYQLKDPRSQDSTLCLFTDFDSQINVPKTMESGTFITDKCVLD
MKAMDSKSNGAIAWSNQTSFTCQDIFKETNATYPSSDVPCDATLTEKSFETDMN
LNFQNLSVMGLRILLLKVAGFNLLMTLRLWSS
SEQ ID NO:12 
ATGGAATCTTTCCTTGGAGGAGTACTGCTCATTTTGTGGCTGCAAGTC
GACTGGGTGAAAAGTCAGAAGATCGAACAGAATAGCGAAGCCCTGAACATC
CAGGAAGGCAAGACTGCTACACTGACTTGTAACTACACAAACTATAGCCCAG
CGTACCTGCAGTGGTACCGGCAGGATCCCGGAAGAGGCCCCGTGTTCCTGC
TGCTTATCAGAGAAAACGAAAAGGAAAAACGAAAGGAGCGGCTGAAGGTTAC
ATTTGATACCACCCTTAAACAGTCCCTGTTTCACATTACTGCAAGTCAGCCAG
CTGACTCCGCAACATATCTGTGCGCCCCCGACAACGCGGGTAATATGCTTAC
GTTTGGCGGAGGTACCAGACTTATGGTAAAGCCGCACATCCAGAATCCTGAG
CCTGCGGTTTACCAGTTGAAAGATCCAAGGTCACAGGACTCCACATTGTGCC
TGTTCACAGACTTCGATTCCCAGATCAATGTGCCTAAGACGATGGAGTCCGG
AACCTTTATTACGGACAAATGCGTGCTGGATATGAAGGCGATGGATAGCAAG
TCAAACGGCGCAATCGCTTGGTCAAACCAGACAAGCTTCACCTGTCAGGATA
TATTCAAAGAGACTAACGCAACCTATCCCTCTTCAGATGTACCATGCGACGCT
ACTTTGACCGAAAAGTCTTTCGAAACCGACATGAACCTGAACTTCCAGAACCT
TTCAGTGATGGGACTTCGCATTTTGCTGCTTAAAGTTGCTGGCTTTAATCTGC
TTATGACATTGCGGCTTTGGTCCTCTATGGATACTTGGCTGGTATGCTGGGC
CATATTCAGTCTGCTCAAAGCCGGACTTACCGAACCTGAAGTGACACAAACT
CCCAGCCACCAAGTGACTCAGATGGGCCAGGAGGTAATCTTGAGGTGTGTG
CCTATCTCCAATCACTTGTACTTCTACTGGTACCGCCAAATTTTGGGGCAAAA
AGTGGAGTTTCTGGTGAGCTTTTATAACAACGAGATCTCTGAAAAATCTGAGA
TCTTCGACGATCAGTTTTCAGTGGAGCGACCCGATGGAAGCAACTTTACGCT
 
TAAAATTCGCAGTACGAAGCTGGAAGACAGCGCAATGTATTTTTGCGCGAGC
AGTGATGATACCGGCGCCAATTACGGTTACACATTCGGCAGCGGCACCCGC
CTCACCGTGGTGGAGGACCTTAGGAACGTGACACCTCCTAAGGTGAGCTTG
TTTGAGCCCAGTAAAGCAGAGATTGCCAATAAGCAGAAGGCTACCCTCGTTT
GCCTCGCCCGGGGATTCTTTCCCGACCATGTTGAACTGAGCTGGTGGGTGA
ATGGGAAGGAAGTTCATAGCGGGGTGTGTACCGATCCACAAGCATACAAAG
AGAGCAACTACAGCTATTGTCTGTCTTCCAGGCTCCGCGTCAGTGCTACGTT
CTGGCACAACCCTCGGAATCATTTCAGGTGTCAGGTTCAGTTTCACGGACTG
TCCGAAGAGGATAAATGGCCTGAAGGAAGTCCTAAGCCTGTTACCCAGAATA
TTAGCGCCGAAGCTTGGGGCAGGGCAGACTGCGGAATCACTAGCGCCTCAT
ACCAGCAAGGCGTGCTGTCCGCTACAATTCTTTATGAGATCCTCTTGGGGAA
GGCCACCCTGTATGCTGTGCTTGTGAGTACACTGGTCGTTATGGCCATGGTG
AAGCGAAAAAATTCT
SEQ ID NO:13 
ATGGACACCTGGCTGGTGTGCTGGGCCATCTTCAGCCTGCTGAAGGC
CGGCCTGACCGAGCCCGAGGTGACCCAGACCCCCAGCCACCAGGTGACCC
AGATGGGCCAGGAGGTGATCCTGAGGTGCGTGCCCATCAGCAACCACCTGT
ACTTCTACTGGTACAGGCAGATCCTGGGCCAGAAGGTGGAGTTCCTGGTGA
GCTTCTACAACAACGAGATCAGCGAGAAGAGCGAGATCTTCGACGACCAGTT
CAGCGTGGAGAGGCCCGACGGCAGCAACTTCACCCTGAAGATCAGGAGCAC
CAAGCTGGAGGACAGCGCCATGTACTTCTGCGCCAGCAGCGACGACACCGG
CGCCAACTACGGCTACACCTTCGGCAGCGGCACCAGGCTGACCGTGGTGGA
GGACCTGAGGAACGTGACCCCCCCCAAGGTGAGCCTGTTCGAGCCCAGCAA
GGCCGAGATCGCCAACAAGCAGAAGGCCACCCTGGTGTGCCTGGCCAGGG
GCTTCTTCCCCGACCACGTGGAGCTGAGCTGGTGGGTGAACGGCAAGGAG
GTGCACAGCGGCGTGTGCACCGACCCCCAGGCCTACAAGGAGAGCAACTAC
AGCTACTGCCTGAGCAGCAGGCTGAGGGTGAGCGCCACCTTCTGGCACAAC
CCCAGGAACCACTTCAGGTGCCAGGTGCAGTTCCACGGCCTGAGCGAGGAG
GACAAGTGGCCCGAGGGCAGCCCCAAGCCCGTGACCCAGAACATCAGCGC
CGAGGCCTGGGGCAGGGCCGACTGCGGCATCACCAGCGCCAGCTACCAGC
AGGGCGTGCTGAGCGCCACCATCCTGTACGAGATCCTGCTGGGCAAGGCCA
CCCTGTACGCCGTGCTGGTGAGCACCCTGGTGGTGATGGCCATGGTGAAGA
GGAAGAACAGCAGGGCCAAGAGGGGCAGCGGCGCCACCAACTTCAGCCTG
CTGAAGCAGGCCGGCGACGTGGAGGAGAACCCCGGCCCCATGGAGAGCTT
 
CCTGGGCGGCGTGCTGCTGATCCTGTGGCTGCAGGTGGACTGGGTGAAGA
GCCAGAAGATCGAGCAGAACAGCGAGGCCCTGAACATCCAGGAGGGCAAG
ACCGCCACCCTGACCTGCAACTACACCAACTACAGCCCCGCCTACCTGCAG
TGGTACAGGCAGGACCCCGGCAGGGGCCCCGTGTTCCTGCTGCTGATCAG
GGAGAACGAGAAGGAGAAGAGGAAGGAGAGGCTGAAGGTGACCTTCGACA
CCACCCTGAAGCAGAGCCTGTTCCACATCACCGCCAGCCAGCCCGCCGACA
GCGCCACCTACCTGTGCGCCCCCGACAACGCCGGCAACATGCTGACCTTCG
GCGGCGGCACCAGGCTGATGGTGAAGCCCGACATCCAGAACCCCGAGCCC
GCCGTGTACCAGCTGAAGGACCCCAGGAGCCAGGACAGCACCCTGTGCCT
GTTCACCGACTTCGACAGCCAGATCAACGTGCCCAAGACCATGGAGAGCGG
CACCTTCATCACCGACAAGTGCGTGCTGGACATGAAGGCCATGGACAGCAA
GAGCAACGGCGCCATCGCCTGGAGCAACCAGACCAGCTTCACCTGCCAGGA
CATCTTCAAGGAGACCAACGCCACCTACCCCAGCAGCGACGTGCCCTGCGA
CGCCACCCTGACCGAGAAGAGCTTCGAGACCGACATGAACCTGAACTTCCA
GAACCTGAGCGTGATGGGCCTGAGGATCCTGCTGCTGAAGGTGGCCGGCTT
CAACCTGCTGATGACCCTGAGGCTGTGGAGCAGC
SEQ ID NO:14 
MESFLGGVLLILWLQVDWVKSQKIEQNSEALNIQEGKTATLTCNYTNYSP
AYLQWYRQDPGRGPVFLLLIRENEKEKRKERLKVTFDTTLKQSLFHITASQPADS
ATYLCAPDNAGNMLTFGGGTRLMVKP
SEQ ID NO:15

MDTWLVCWAIFSLLKAGLTEPEVTQTPSHQVTQMGQEVILRCVPISNHLY
FYWYRQILGQKVEFLVSFYNNEISEKSEIFDDQFSVERPDGSNFTLKIRSTKLEDS
AMYFCASSDDTGANYGYTFGSGTRLTVV

### Example 1: Sorting of TP53-R248Q-TCR-T Cells and TCR Sequencing

### 1. In vitro induction of monocytes from a healthy donor into dendritic cells (DCs)

(1) Peripheral blood mononuclear cells (PBMCs) were obtained from the blood of a healthy donor or patient by Lymphoprep density-gradient centrifugation, or were obtained by thawing previously cryopreserved PBMCs. The PBMCs were resuspended in 10 mL of 1640 medium (Gibco), centrifuged at 350 × g for 5 min, and the supernatant was removed. The cells were resuspended in an appropriate volume of DC medium (Stemcell Technologies) to a final density of 5×10⁶ cells/mL.
(2) 2 mL of the PBMC suspension described above was transferred into each well of a 6-well plate, corresponding to 1×10⁷ cells per well. The 6-well plate was incubated at 37°C within a 5% CO₂ incubator for 2 h. The 6-well plate was gently rotated until a thin layer of non-adherent cells was observed floating in the center of each well. The plate was gently tilted, and the culture medium, together with clumped non-adherent cells, was carefully aspirated without disturbing the adherent cells.
(3) Subsequently, 3 mL of DC medium (Stemcell Technologies) containing DC Differentiation Supplement at a final concentration of 1× was gently added along the side wall of each well. The 6-well plate was incubated at 37°C within a 5% CO₂ incubator for 3 days.
(4) On Day 5, without replacing the culture medium, 30 µL of 100× DC Maturation Supplement (Stemcell Technologies) was added to each well. The 6-well plate was further incubated at 37°C within a 5% CO₂ incubator for 2 days to induce maturation.
(5) The cells at the bottom of each well were gently pipetted to resuspend the cells, and mature dendritic cells (mature DCs) were collected. The collected mature DCs were transferred into a 15-mL centrifuge tube. The bottom of the wells was examined under a microscope to ensure that all mature DCs had been completely collected.

### 2. Co-stimulation culture of DCs loaded with TP53-R248Q antigen peptide and autologous CD8⁺ T cells

(1) 5 µg/mL TP53-R248Q target peptide was added to the 15-mL centrifuge tube containing the mature DCs. The tube was sealed with Parafilm and placed on a MACSmix rotator, followed by incubation at 37°C within a 5% CO₂ incubator for 4 h to allow peptide loading.
(2) PBMCs were obtained from the blood of a healthy donor or patient by Lymphoprep density-gradient centrifugation, or were obtained by thawing previously cryopreserved PBMCs. The PBMCs were resuspended in 10 mL of 1640 medium, centrifuged at 350 × g for 8 min, and the supernatant was removed. CD8⁺ T cells were isolated from PBMCs using the EasySep^{™} Human CD8 Positive Selection Kit.
(3) After completion of peptide loading, the TP53-R248Q-peptide-loaded mature DCs were irradiated at 4000 rad (40 Gy) using an irradiator.
(4) After irradiation, the TP53-R248Q-peptide-loaded mature DCs were co-cultured with autologous CD8⁺ T cells at a ratio of DCs to CD8⁺ T cells of 1:2.5 to stimulate the CD8⁺ T cells *in vitro.* The co-stimulation culture was plated in a 12-well plate at 2 mL per well, and incubated overnight at 37°C within a 5% CO₂ incubator.
(5) Subsequently, half of the culture medium was replaced every 2-3 days. On Day 14, freshly prepared irradiated mature DCs loaded with TP53-R248Q peptide were added to the co-stimulation culture described in step (4) for a second round of *in vitro* stimulation.
(6) On Day 28, cells from the co-stimulation culture were collected for fluorescence-activated cell sorting (FACS) staining. Specifically, 10 µL of HLA-A*11:01-TP53-R248Q-tetramer-PE was added, mixed thoroughly, and incubated at room temperature for 20 min in the dark. Subsequently, 5 µL of CD8a-APC was added, mixed, and incubated at 4°C for 20 min in the dark. After incubation, the cells were washed three times with FACS buffer, resuspended in 400 µL of FACS buffer containing 1× DAPI working solution, and transferred into FACS tubes. After setting appropriate single-color controls, unstained controls, and isotype controls, flow cytometric analysis was performed to determine the percentage of CD8⁺/tetramer⁺ cells.

### 3. Sorting of TP53-R248Q-specific T cells and single-cell TCR sequencing

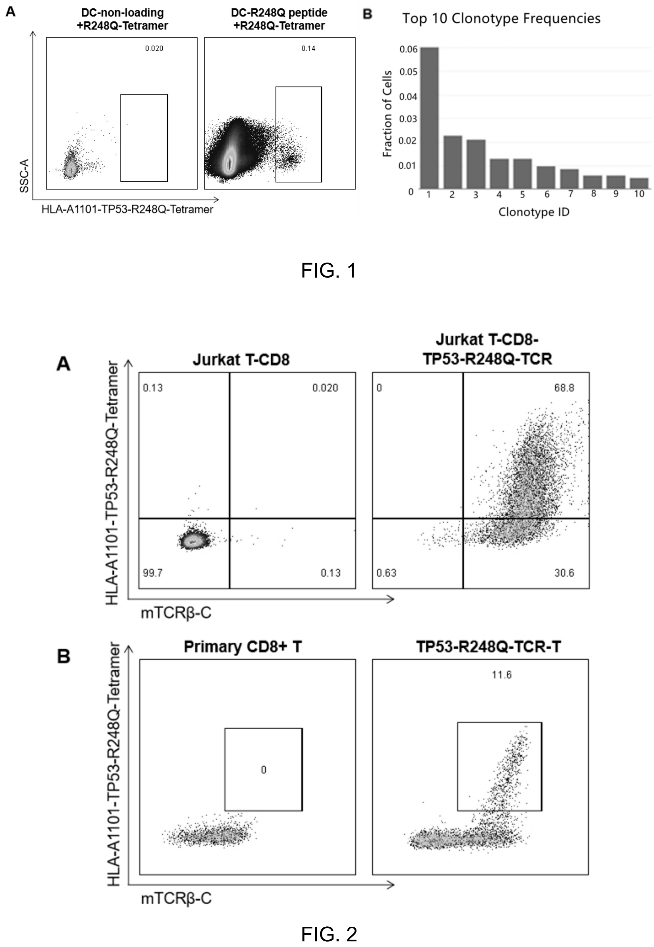

(1) CD8⁺ T cells from a healthy donor were *in vitro* stimulated with autologous TP53-R248Q-peptide-loaded DCs for two rounds, and the resulted cells were collected and analyzed by flow cytometry. Representative flow cytometry results are shown in panel A of FIG. 1. The proportion of CD8a⁺/tetramer⁺ T cells was 0.14%, with a clearly distinguishable population, indicating that TP53-R248Q-specific TCR-T cells were specifically expanded during the two rounds of *in vitro* stimulation. CD8⁺/tetramer⁺ T cells were subsequently specifically sorted from the co-culture system using a flow cytometric cell sorter. The sorted cells were then sent to Genergy Bio-Technology Co., Ltd. for 10× Genomics single-cell TCR and transcriptome sequencing, to obtain TCR sequences and transcriptomic characteristics of the CD8⁺/tetramer⁺ T cells.
(2) After completion of single-cell sequencing, transcriptomic characteristic analysis was performed on the top four ranked TCRs. The TCR sequencing results and frequency rankings of this cell population are shown in panel B of FIG. 1. The TCR sequence with the highest frequency was further optimized by replacing its TCR constant regions (TCR-C regions) with mTCR constant regions (mTCR-C regions), including mTCRα-C and mTCRβ-C, to prevent TCR mispairing. The amino acid sequence of mTCRα-C is as set forth in SEQ ID NO:11, and the amino acid sequence of mTCRβ-C is as set forth in SEQ ID NO:10. The obtained TCR after optimization included a TCRα chain amino acid sequence as set forth SEQ ID NO:8 and a TCRβ chain amino acid sequence as set forth in SEQ ID NO:7. The amino acid sequences of CDRα1, CDRα2, and CDRα3 of the TCRα chain are as set forth in SEQ ID NOs:3, 4, and 2, respectively. The amino acid sequences of CDRβ1, CDRβ2, and CDRβ3 of the TCRβ chain are as set forth in SEQ ID NOs:5, 6, and 1, respectively. The TCR sequences were further optimized by human codon optimization to enhance TCR expression. The resulting nucleotide sequence of the DNA strand encoding the TCR is as set forth in SEQ ID NO:12. A P2A peptide and a furin cleavage site were used to enable simultaneous expression of the TCR α and β chains in a lentiviral expression vector. The nucleotide sequence of the DNA strand encoding the protein expressed by the lentiviral expression vector is as set forth in SEQ ID NO:13.

### Example 2: In Vitro Binding Assay of TP53-R248Q-TCR-T Cells

### 1. Preparation of TP53-R248Q-TCR-T Cells

(1) The lentiviral expression vector obtained in Example 1 was constructed, and the vector was subsequently packaged into lentiviral particles. The lentiviral packaging procedure was performed as follows. 293T cells were trypsinized and passaged, and cell density was examined on the day of transfection. When the cells reached approximately 80-90% confluence, transfection was performed without replacing the culture medium. Opti-MEM was prewarmed in a 37°C water bath before use. Fugene-HD transfection reagent was equilibrated to room temperature and mixed well before use. For each T75 flask, the transfection complex contained the following components: 10 µg of pHR-SFFV-TCR-Puro plasmid encoding the target TCR, 5 µg of pMD2.G (envelope plasmid), and 7.5 µg of pSPAX2 (packaging plasmid). Opti-MEM was added to a final volume of 1 mL and mixed thoroughly, then 56 µL Fugene-HD transfection reagent was added. The mixture was gently pipetted 5-6 times, incubated at room temperature for 15 min, and then added to the T75 flask. Viral supernatants were collected at 48 h and 72 h post-transfection. The collected supernatants were centrifuged at 4000 rpm for 5 min and filtered through a 0.45 µm PES membrane to obtain TP53-R248Q-TCR lentiviral preparations.
(2) Jurkat T cells were transduced with TP53-R248Q-TCR lentiviral particles in the presence of 10 µg/mL polybrene and subjected to spin infection at 800 × g for 90 min at 30°C. After centrifugation, the Jurkat T cells were cultured at 37°C within a 5% CO₂ incubator for 24 h, followed by replacement with fresh 1640 medium.
(3) CD8⁺ T cells from a healthy donor were activated by adding 20 µL of CD3/CD28 beads (GenScript) and 20 ng/mL IL-2. After 48 h of activation, 10 µg/mL polybrene was added, and a first round of TP53-R248Q-TCR lentiviral transduction was performed. After centrifugation, the cells were cultured at 37°C within a 5% CO₂ incubator for 24 h, followed by a second round of TP53-R248Q-TCR lentiviral transduction.

### 2. FACS Analysis of Binding Specificity of TP53-R248Q-TCR-T Cells

The TP53-R248Q-TCR-T cells prepared as described above were stained with mTCRβC⁺ and HLA-A*11:01-TP53-R248Q-tetramer and analyzed by flow cytometry. The flow cytometry results showed clearly distinguishable populations of tetramer⁺/mTCRβC⁺ Jurkat T cells (panel A of FIG. 2) and CD8⁺/tetramer⁺ primary CD8⁺ T cells (panel B of FIG. 2). These results demonstrated that the TP53-R248Q-TCR identified and characterized in this *in vitro* screening could be properly expressed in both the Jurkat T cell line and primary CD8⁺ T cells, and could specifically bind to the HLA-A*11:01-TP53-R248Q tetramer.

### Example 3: In Vitro Activation Assay of TP53-R248Q-TCR-T Cells

The *in vitro* activation of TP53-R248Q-TCR-T cells was evaluated using an IFN-y ELISpot assay, which included the following steps.
(1) Autologous monocytes from a donor were differentiated into DCs *in vitro.* The DCs separately loaded with TP53-R248-WT or TP53-R248Q antigen peptides were prepared as described in Example 1. The TP53-R248Q-TCR-T cells prepared in Example 2 were co-cultured *in vitro* with DCs loaded with either TP53-R248-WT or TP53-R248Q antigen peptides.
(2) After 24 h of co-culture, the cells were discarded, and the wells were washed five times with 200 µL DPBS to completely remove cells. Subsequently, 100 µL of detection antibody 7-B6-1-biotin (1 µg/mL) was added to each well, followed by incubation at 37°C for 2 h. Then, 100 µL of streptavidin-HRP (3420-2H, Mabtech) was added and incubated at room temperature for 1 h.
(3) The wells were washed five times with 200 µL DPBS, and 100 µL of TMB substrate was added to each well until distinct spots were observed. The color development reaction was then stopped by adding sterile water.

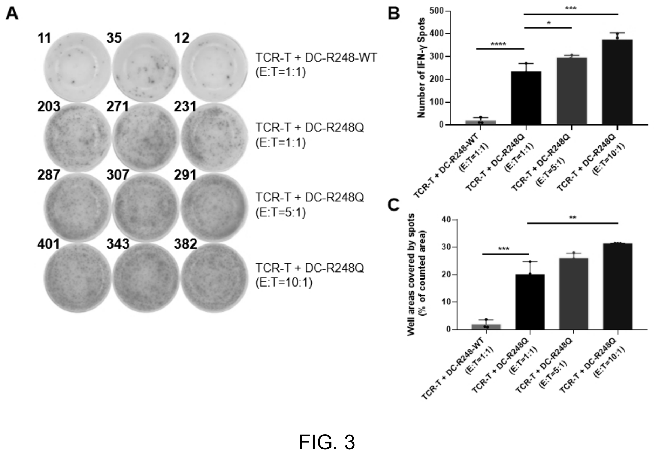

As shown in FIG. 3, DCs loaded with the TP53-R248Q antigen peptide specifically activated TP53-R248Q-TCR-T cells *in vitro.* Following activation, IFN-y expression levels of TP53-R248Q-TCR-T cells were significantly increased (panels A-C of FIG. 3). In contrast, DCs loaded with the TP53-R248-WT antigen peptide did not induce significant activation of TP53-R248Q-TCR-T cells (panels B and C of FIG. 3). These results demonstrated that the TP53-R248Q-TCR identified and characterized herein specifically recognizes the TP53-R248Q antigen peptide.

### Example 4: In Vitro Cytotoxicity Assay of TP53-R248Q-TCR-T Cells

The *in vitro* cytotoxicity of TP53-R248Q-TCR-T cells was evaluated using an Incucyte-based killing assay, which included the following steps.
(1) T cells or K562-A1101 cells were transduced *in vitro* with lentiviral vectors to generate TP53-R248Q-TCR-T effector cells and K562-A*11:01-TP53-R248Q target cells, respectively. Then, TP53-R248Q-TCR-T cells or CD8⁺ T cells were co-cultured with K562-A*11:01 control cells or K562-A*11:01-TP53-R248Q target cells in 96-well plates at effector-to-target (E: T) ratios of 1:1, 2:1, or 5:1.
(2) Incucyte^{®} Annexin V Dyes (Sartorius) were dissolved in 100 µL DPBS. The mixture was subsequently diluted in complete culture medium to a final dilution of 1:200.
(3) The 96-well plates containing the co-cultured cells were placed into an Incucyte^{®} (Sartorius) live-cell analysis system, and apoptosis was monitored using appropriate fluorescence channels. The parameters were set as follows: (A) objective magnification: 20×; (B) fluorescence channel: phase contrast, Green, Red, Orange, and near-infrared (NIR); (C) scan type: 2-4 images per well per scan; and (D) scan interval: once every 30 min, until cytotoxicity assessment was completed after 48 h of co-culture.
(4) After completion of the assay, the *in vitro* killing efficiency of TP53-R248Q-TCR-T cells was analyzed using the Incucyte^{®} live-cell analysis software (Sartorius).

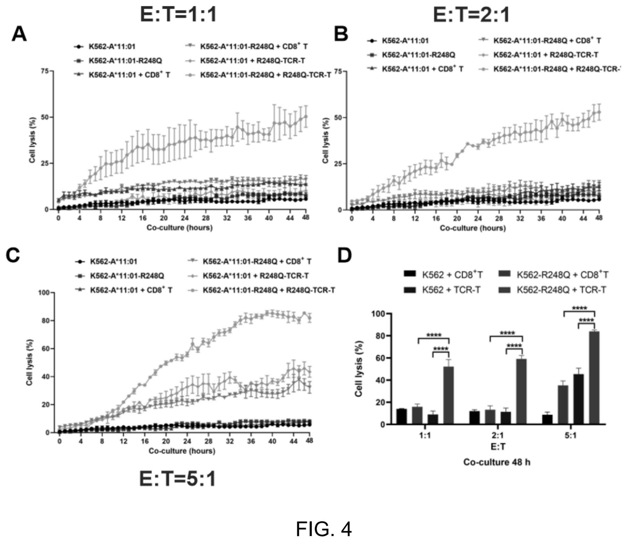

As shown in FIG. 4, the Incucyte high-throughput, long-term dynamic imaging and analysis system was used to monitor, in real time, the cytotoxic activity of TP53-R248Q-TCR-T cells against target cells. The results showed that within 48 h of co-culture, compared with negative control CD8⁺ T cells not transduced with TP53-R248Q-TCR, TP53-R248Q-TCR-T cells at different E:T ratios (1:1, 2:1, and 5:1) exhibited significantly more efficient killing of HLA-A*11:01-positive target cells expressing the TP53-R248Q mutation (as shown in panels A-D of FIG. 4).

### Example 5: In Vivo Cytotoxicity Assay of TP53-R248Q-TCR-T Cells

The *in vivo* cytotoxicity of TP53-R248Q-TCR-T cells was evaluated using a Cell line-Derived Xenograft (CDX) animal model, which included the following steps.
(1) Female NCG mice (GemPharmatech), aged 6-8 weeks, were selected, and 3×10⁶ HLA subtype-matched K562 cells expressing the TP53-R248Q mutant protein and a luciferase reporter gene were injected into the mice via the lateral tail vein.
(2) Three days after transplantation of the K562-HLA-TP53-R248Q-luciferase cells, 1×10⁷ TCR-T cells were injected via the lateral tail vein. Mice injected with T lymphocytes not transduced with the TCR gene were used as a negative control.
(3) Beginning after tumor cell inoculation, *in vivo* bioluminescent imaging was performed every 7 days. Body weight changes and survival of the mice were also monitored to evaluate the *in vivo* cytotoxic effect of TP53-R248Q-TCR-T cells against target cells.

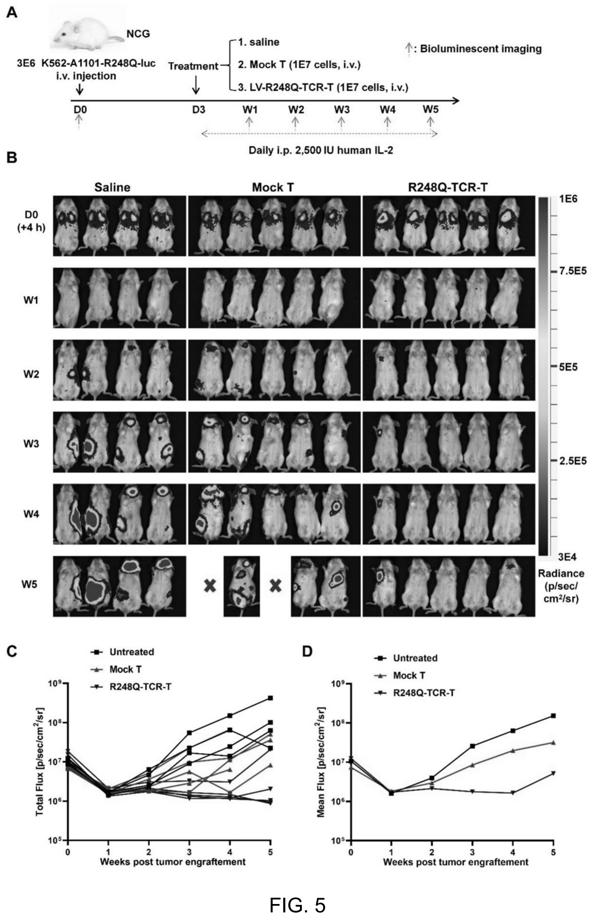

As shown in FIG. 5, a CDX model was used to evaluate the *in vivo* killing efficacy of TP53-R248Q-TCR-T cells against target cells. On day 0, 3×10⁶ K562-HLA-A*11:01-R248Q-luciferase cells were injected via the lateral tail vein into immunodeficient NCG mice. On day 3, 1×10⁷ mock T cells or TP53-R248Q-TCR-T cells were adoptively transferred, respectively. After T-cell infusion, 2500 IU of human IL-2 was administered daily by intraperitoneal injection. Bioluminescent imaging was performed at 4 h and at weeks 1-5 after tumor cell inoculation (panel A of FIG. 5). The *in vivo* imaging results showed that mice in the saline group and the mock T-cell group exhibited obvious tumor growth within 5 weeks after tumor cell inoculation, whereas a portion of the mice (3/5) in the TP53-R248Q-TCR-T group showed no obvious tumor growth (panel B of FIG. 5). In addition, both the total photon flux and the average photon intensity of luciferase signals in the TP53-R248Q-TCR-T group were significantly lower than those in the saline group and the mock T-cell control group (panels C and D of FIG. 5). These CDX experimental results demonstrated that under HLA-A*11:01-restriction TP53-R248Q-TCR-T cells were capable of efficiently and specifically killing K562-HLA-A*11:01-R248Q-luciferase target cells *in vivo.*

The foregoing examples are provided for illustrating the embodiments of the present disclosure and are not to be construed as limiting the present disclosure. In addition, various modifications and variations of the methods described herein will be apparent to those skilled in the art without departing from the scope of the claims. Although the present disclosure has been described with reference to multiple preferred embodiments, it should be understood that the invention is not limited to these specific embodiments. Indeed, all modifications that are obvious to those skilled in the art should be included within the scope of the present disclosure.

## Claims

1. An isolated T cell receptor (TCR), comprising a TCR α chain and a TCR β chain;
wherein the TCR α chain comprises CDRα1, CDRα2, and CDRα3 within a variable region of the TCR α chain, and the TCR β chain comprises CDRβ1, CDRβ2, and CDRβ3 within a variable region of the TCR β chain; and
wherein CDRα3 comprises an amino acid sequence as set forth in SEQ ID NO. 2, and CDRβ3 comprises an amino acid sequence as set forth in SEQ ID NO. 1.

2. The TCR according to claim 1, wherein CDRα1 comprises an amino acid sequence as set forth in SEQ ID NO. 3, or CDRα2 comprises an amino acid sequence as set forth in SEQ ID NO. 4;
and/or CDRβ1 comprises an amino acid sequence as set forth in SEQ ID NO. 5, or CDRβ2 comprises an amino acid sequence as set forth in SEQ ID NO. 6.

3. The TCR according to claim 1, wherein the variable region of the TCR α chain comprises an amino acid sequence as set forth in SEQ ID NO. 14; and/or the variable region of the TCR β chain comprises an amino acid sequence as set forth in SEQ ID NO. 15.

4. The TCR according to claim 1, wherein the TCR α chain comprises an amino acid sequence as set forth in SEQ ID NO. 8; and/or the TCR β chain comprises an amino acid sequence as set forth in SEQ ID NO. 7.

5. A polynucleotide, wherein the polynucleotide encodes the TCR according to any one of claims 1-4.

6. The polynucleotide according to claim 5, wherein the polynucleotide comprises a nucleotide sequence as set forth in SEQ ID NO. 9.

7. A nucleic acid construct, comprising the polynucleotide according to any one of claims 5-6 and a plasmid backbone.

8. The nucleic acid construct according to claim 7, wherein the plasmid backbone is a lentiviral, adenoviral, or adeno-associated viral plasmid backbone; preferably, the plasmid backbone is selected from the group consisting of pLKO.1-CMV-tGFP, pLKO.1-puro-CMV-tGFP, pLKO.1-CMV-Neo, pLKO.1-Neo, pLKO.1-Neo-CMV-tGFP, pLKO.1-puro-CMV-TagCFP, pLKO.1-puro-CMV-TagYFP, pLKO.1-puro-CMV-TagRFP, pLKO.1-puro-CMV-TagFP635, pLKO.1-puro-UbC-TurboGFP, pLKO.1-puro-UbC-TagFP635, pLKO-puro-IPTG-1xLacO, pLKO-puro-IPTG-3xLacO, pLP1, pLP2, pLP/VSV-G, pENTR/U6, pLenti6/BLOCK-iT-DEST, pcDNA1.2/V5-GW/lacZ, pLenti6.2/N-Lumio/V5-DEST, pGCSIL-GFP, pHR-SFFV-Puro, and any combination thereof.

9. A virus, comprising the polynucleotide according to any one of claims 5-6 or the nucleic acid construct according to any one of claims 7-8.

10. A T cell, comprising the TCR according to any one of claims 1-4, the polynucleotide according to any one of claims 5-6, the nucleic acid construct according to any one of claims 7-8, or the virus according to claim 9.

11. The T cell according to claim 10, wherein the T cell is a TCR-T cell;
and/or the T cell is selected from the group consisting of helper T cells, suppressor T cells, effector T cells, cytotoxic T cells, delayed-type hypersensitivity T cells, natural T cells, and memory T cells;
and/or the T cell is a Jurkat T cell or a CD8⁺ T cell.

12. A use of the TCR according to any one of claims 1-4, the polynucleotide according to any one of claims 5-6, the nucleic acid construct according to any one of claims 7-8, the virus according to claim 9, or the T cell according to any one of claims 10-11, in the manufacture of a tumor therapeutic product.

13. The use according to claim 13, wherein the tumor is one or more of adrenocortical carcinoma, urothelial carcinoma (bladder cancer), breast cancer, cervical squamous cell carcinoma, cervical adenocarcinoma, cholangiocarcinoma, colon adenocarcinoma, lymphoid neoplasm, esophageal carcinoma, glioblastoma multiforme, head and neck squamous cell carcinoma, chromophobe renal cell carcinoma, clear cell renal cell carcinoma, papillary renal cell carcinoma, acute myeloid leukemia, low-grade glioma, hepatocellular carcinoma, mesothelioma, ovarian cancer, pancreatic cancer, pheochromocytoma, paraganglioma, prostate cancer, rectal cancer, sarcoma, melanoma, gastric cancer, testicular germ cell tumor, thyroid carcinoma, thymic carcinoma, endometrial carcinoma, chronic myeloid leukemia, lung cancer, anal cancer, and retinoblastoma.

14. A tumor therapeutic product, comprising the TCR according to any one of claims 1-4, the polynucleotide according to any one of claims 5-6, the nucleic acid construct according to any one of claims 7-8, the virus according to claim 9, or the T cell according to any one of claims 10-11, and a pharmaceutically acceptable carrier.

15. A method for tumor treatment, comprising administering to a subject in need thereof an effective amount of the TCR according to any one of claims 1-4, the polynucleotide according to any one of claims 5-6, the nucleic acid construct according to any one of claims 7-8, the virus according to claim 9, the T cell according to any one of claims 10-11, or the therapeutic product according to claim 14.

16. The method according to claim 15, further comprising one or more of the following:
(1) an administered amount is in a range of 1-1000 mg/kg/day;
(2) the subject is a mammal; preferably, the subject is a human;
(3) the tumor is a tumor in which a wild-type TP53 gene harbors an R248Q mutation.
